# EUROPEAN PATENT APPLICATION

(11) **EP 4 115 827 A1**
(43) Date of publication of application: **11.01.2023**
(21) Application number: 20964946.6
(22) Date of filing: 28.12.2020
(51) Int. Cl.: A61B 18/12

(54) **PERFUSION CONTROL METHOD, APPARATUS AND SYSTEM FOR SYRINGE PUMP, AND COMPUTER-READABLE STORAGE MEDIUM**

(30) Priority: 09.12.2020 CN 202011433510
(71) Applicant: Hangzhou Broncus Medical Co., Ltd., Hangzhou, Zhejiang 310051 (CN)
(72) Inventor: XU, Hong, Hangzhou, Zhejiang 310051 (CN); CUI, Changjie, Hangzhou, Zhejiang 310051 (CN)
(74) Representative: MERH-IP Matias Erny Reichl Hoffmann Patentanwälte PartG mbB
(86) International application number: PCT/CN2020/140380
(87) International publication number: WO 2022/121017

(57) **Abstract**

A perfusion control method, apparatus and system for syringe pump, and a computer-readable storage medium, wherein the method includes: when an ablation task is triggered, controlling the syringe pump to execute a perfusion operation on an ablation object, and acquiring an impedance value and/or a temperature value of the ablation object in real time; analyzing the acquired impedance value and/or temperature value to obtain real-time change information of the impedance value and/or temperature value; and adjusting a perfusion amount of the syringe pump dynamically according to the real-time change information obtained from the analysis. By means of the perfusion control method, apparatus and system for syringe pump, and the computer-readable storage medium, dynamic adjustment of the perfusion amount of the syringe pump can be achieved, thereby improving the timeliness and accuracy of liquid perfusion during the process of executing an ablation task.

## Description

### TECHNICAL FIELD

Embodiments of the present application relate to the technical field of communication, and particularly to a perfusion control method, apparatus and system for syringe pump, and a non-transitory computer-readable storage medium.

### BACKGROUND

Lung cancer is one of the most common malignant tumors. During clinical treatment, surgical resection is still the first choice for the treatment of early lung cancer. However, for lung cancer patients who are old, weak, poor in cardiopulmonary function, or have complications, they are not suitable or intolerant to conventional surgical resection. Therefore, local treatment methods such as minimally invasive tumor ablation are developed.

Radio frequency ablation (RFA) is a relatively common method of minimally invasive tumor ablation. The principle of the radio frequency ablation is to apply alternating high-frequency currents with a frequency less than 30MHz (megahertz) to make ions in tumor tissues oscillate at a high speed, rub each other to convert radio frequency energy into heat energy, thereby causing coagulative necrosis of tumor cells.

In an ablation process, the temperature of the human body will change under the influence of the alternating high-frequency currents. Once the temperature exceeds a normal value, it will cause irreversible damage to the human body. Therefore, it is necessary to perfuse an ablation site with physiological saline in the ablation process.

### TECHNICAL PROBLEM

In the prior art, a perfusion operation of the physiological saline is generally controlled manually. When and how much liquid needs to be perfused completely depends on the treatment experience and the response ability of the doctor. As such, misjudgments and delayed operations are common. Therefore, how to improve the timeliness and accuracy of liquid perfusion of a radio frequency ablation perfusion device is a major problem to be solved in the industry.

### SUMMARY

Embodiments of the present application provide a perfusion control method, apparatus and system for syringe pump and a non-transitory computer-readable storage medium, which may realize dynamic adjustment of the perfusion amount of the syringe pump, improving the timeliness and accuracy of liquid perfusion during the process of executing an ablation task.

On one aspect, the embodiments of the present application provides a perfusion control method for syringe pump, including:
when an ablation task is triggered, controlling the syringe pump to execute a perfusion operation on an ablation object, and acquiring an impedance value and/or a temperature value of the ablation object in real time;
analyzing the acquired impedance value and/or temperature value to obtain real-time change information of the impedance value and/or temperature value; and
adjusting a perfusion amount of the syringe pump dynamically according to the real-time change information obtained from the analysis.

On one aspect, the embodiments of the present application further provides a perfusion control apparatus for syringe pump, including:
a control module configured for controlling the syringe pump to execute a perfusion operation on an ablation object when an ablation task is triggered, and acquiring an impedance value and/or a temperature value of the ablation object in real time;
an analysis module configured for analyzing the acquired impedance value and/or temperature value to obtain real-time change information of the impedance value and/or temperature value; and
an adjustment module configured for adjusting the perfusion amount of the syringe pump dynamically according to the real-time change information obtained from the analysis.

On one aspect, the embodiments of the present application further provides an electronic apparatus, including a non-transitory memory and a processor, wherein
the non-transitory memory stores an executable program code;
the processor is electrically coupled to the non-transitory memory, a temperature acquisition device and an impedance acquisition device;
the processor calls the executable program code stored in the non-transitory memory to execute the perfusion control method for syringe pump provided by the above embodiments.

On one aspect, the embodiments of the present application further provides a perfusion control system for syringe pump, including a syringe pump, a temperature acquisition device and an impedance acquisition device, wherein
the syringe pump includes a controller, a syringe, a push rod and a driving device, wherein
the controller is electrically coupled to the driving device, the temperature acquisition device and the impedance acquisition device, for executing the steps of the perfusion control method for syringe pump provided by the above embodiments;
the driving device is configured for driving the push rod to move along a desired direction with a desired speed pointed by the control instruction of the controller so as to control and adjust the perfusion amount of the syringe;
the temperature acquisition device is configured for acquiring a temperature value of the ablation object and transmitting it to the controller; and
the impedance acquisition device is configured for acquiring an impedance value of the ablation object and transmitting it to the controller.

On one aspect, the embodiments of the present application further provides a radio frequency ablation system, including a radio frequency ablation control apparatus, a radio frequency ablation catheter, a neutral electrode, a syringe pump, a temperature acquisition device and an impedance acquisition device, wherein
the radio frequency ablation control apparatus is configured for executing steps of the perfusion control method for syringe pump provided by the above embodiments;
the radio frequency ablation catheter is configured for executing an ablation operation on the ablation object according to a control instruction of the radio frequency ablation control apparatus.
the temperature acquisition device is configured for acquiring a temperature value of the ablation object and transmitting it to the radio frequency ablation control apparatus; and
the impedance acquisition device is configured for acquiring an impedance value of the ablation object and transmitting it to the radio frequency ablation control apparatus.

The embodiments of the present application further provides a non-transitory computer-readable storage medium on which a computer program is stored, wherein the computer program, when executed by a processor, implements the above perfusion control method for syringe pump.

### BENEFICIAL EFFECT

When the ablation task is triggered, the syringe pump is controlled to execute the perfusion operation on the ablation object, and the impedance value and/or temperature value of the ablation object are acquired in real time; and then the acquired impedance value and/or temperature value are/is analyzed, and the perfusion amount of the syringe pump is dynamically adjusted according to real-time change information of the impedance value and/or temperature value obtained from the analysis. Accordingly, automatic, dynamic adjustment of the perfusion amount of the syringe pump is realized based on the analysis of changes of the impedance value and/or temperature value of the ablation object during the process of executing the ablation task. Since the perfusion amount of the syringe pump is adjusted automatically and dynamically along with the changing of the impedance value and/or temperature value of the ablation object, operation delays and errors caused by manual determination can be reduced, and the timeliness and accuracy of liquid perfusion during the process of executing the ablation task can be improved. Accordingly, the injury of the ablation operation to the ablation object is reduced, and the safety of the radio frequency ablation treatment is improved.

### BRIEF DESCRIPTION OF THE DRAWINGS

In order to describe the technical solutions according to the embodiments of the present application or in the prior art more clearly, the drawings needed to be used in the embodiments or in the prior art will be described briefly below. Apparently, the drawings in the following description show some embodiments of the present application. Other drawings can be obtained by persons of ordinary skill in the art based on these drawings without creative efforts.
FIG. 1 is a diagram of an application sense of a perfusion control method for syringe pump provided by an embodiment of the present application;
FIG. 2 is a schematic diagram of a perfusion control system for syringe pump provided by an embodiment of the present application;
FIG. 3 is a schematic diagram of a syringe pump of the perfusion control system for syringe pump showing in FIG. 2;
FIG. 4 is a flow chart of the perfusion control method for syringe pump provided by an embodiment of the present application;
FIG. 5 is a flow chart of the perfusion control method for syringe pump provided by another embodiment of the present application;
FIG. 6 is a flow chart of the perfusion control method for syringe pump provided by another embodiment of the present application;
FIG. 7 is a flow chart of steps S603 to S605 of the embodiment shown in FIG. 6;
FIG. 8 is a flow chart of step S604 of the embodiment shown in FIG. 6;
FIG. 9 is a flow chart of step S605 of the embodiment shown in FIG. 6;
FIG. 10 is a schematic diagram of a perfusion control apparatus for syringe pump provided by an embodiment of the present application;
FIG. 11 is a schematic diagram of a hardware structure of an electronic apparatus provided by an embodiment of the present application; and
FIG. 12 is a schematic diagram of a radio frequency ablation system provided by an embodiment of the present application.

### DESCRIPTION OF THE EMBODIMENTS

In order to make the objects, technical solutions and advantages of the embodiments of the present application clearer, the technical solutions according to the embodiments of the present application will be clearly and completely described with reference to drawings in the embodiments of the present application. Apparently, the embodiments described are merely some embodiments, but not all of the embodiments of the present application. All other embodiments obtained by ordinary persons skilled in the art based on the embodiments of the present application without creative efforts shall fall within the protection scope of the present application.

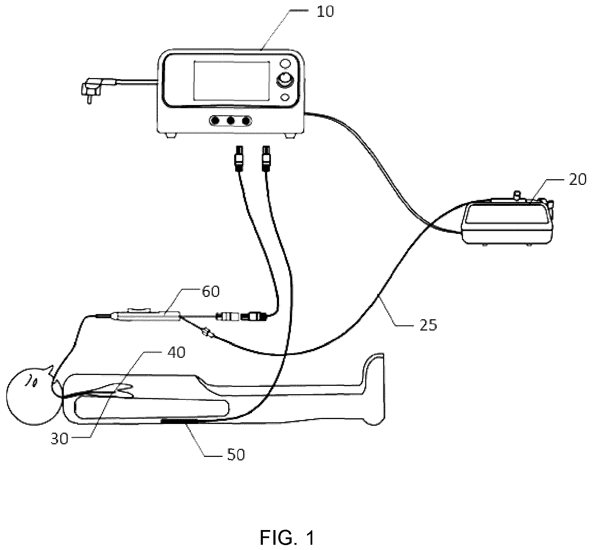

Referring to FIG. 1, which is a diagram of an application sense of a perfusion control method for syringe pump provided by an embodiment of the present application. The perfusion control method for syringe pump may be implemented by a radio frequency ablation control apparatus 10 or a syringe pump 20 shown in FIG. 1. Optionally, the perfusion control method for syringe pump may be implemented by other computer devices other than the radio frequency ablation control apparatus 10 or the syringe pump 20, such as a server, a desktop computer, a notebook computer, a laptop computer, a tablet computer, a personal computer and a smart phone.

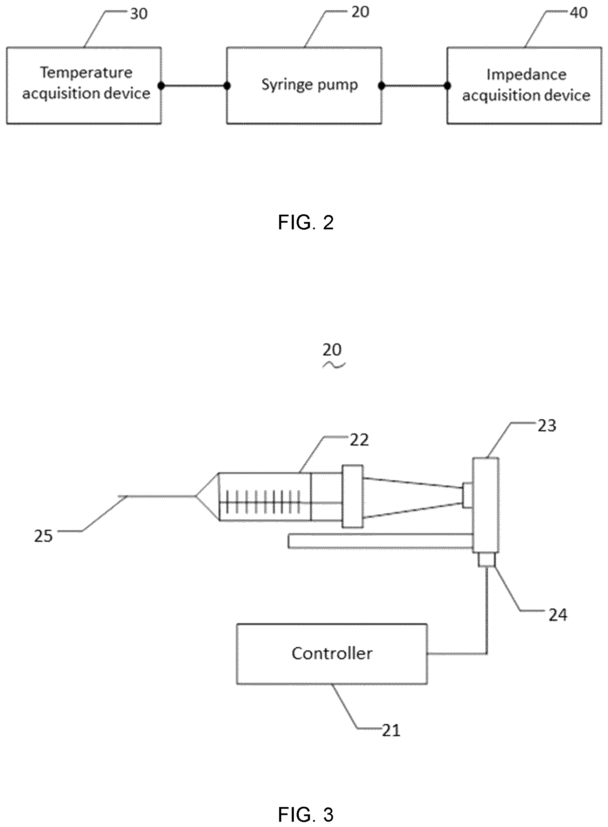

Particularly, when the perfusion control method for syringe pump is implemented by the syringe pump 20, reference is made to FIG. 2 and FIG. 3, wherein FIG. 2 is a schematic diagram of a perfusion control system for syringe pump provided by an embodiment of the present application, and FIG. 3 is a schematic diagram of a syringe pump of the perfusion control system for syringe pump. As shown in FIG. 2, the perfusion control system for syringe pump includes a syringe pump 20, a temperature acquisition device 30 and an impedance acquisition device 40. The temperature acquisition device 30 and the impedance acquisition device 40 are electrically coupled to the syringe pump 20.

As shown in FIG. 3, the syringe pump 20 includes a controller 21, a syringe 22, a push rod 23 and a driving device 24. For facilitating the description, FIG. 3 only shows parts related to the embodiments of the present application.

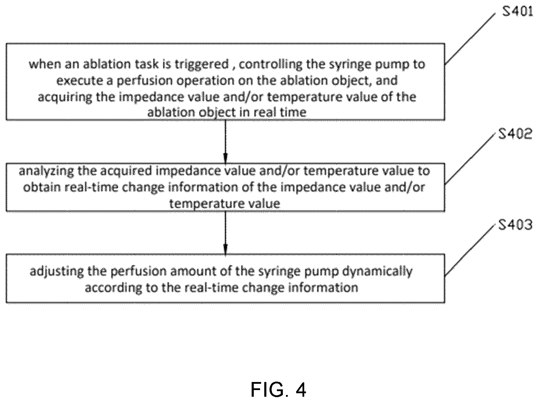

The temperature acquisition device 30 and the impedance acquisition device 40 are electrically coupled to the controller 21. The temperature acquisition device 30 is configured for acquiring a temperature value of an ablation object and transmitting it to the controller 21. The impedance acquisition device 40 is configured for acquiring an impedance value of the ablation object and transmitting it to the controller 21. The controller 21 is configured for executing steps of the perfusion control method for syringe pump provided in the embodiments of FIG. 4 to FIG. 9, so as to realize dynamic adjustment of the perfusion amount of the syringe pump.

The driving device 24 is configured to drive the push rod 23 to move along a desired direction with a desired speed according to a control instruction of the controller 21, so as to control and adjust the perfusion amount of the syringe 22.

Further, the syringe pump 20 includes an extension tube 25. As shown in FIG. 1, one end of the extension tube 25 is connected to a needle of the syringe 22, and the other end is inserted into the ablation object and close to an ablation site.

Optionally, the temperature acquisition device 30 and the impedance acquisition device 40 may be arranged at one end of the extension tube 25 close to the ablation site. Alternatively, the temperature acquisition device 30 and the impedance acquisition device 40 may be arranged on other apparatuses, inserted into the ablation object through the other apparatuses and snuggled onto the ablation site.

In combination with FIG. 1 to FIG. 3, in practical applications, firstly, a radio frequency ablation catheter 60, which is configured to generate and output radio frequency energy, and the extension tube 25 are inserted into the ablation object (such as a lung cancer patient) and reach the ablation site. Then, a neutral electrode 50 is brought into contact with the skin surface of the ablation object. A radio frequency current flows through the radio frequency ablation catheter 60, tissues of the patient and the neutral electrode 50, thereby forming a loop. When the ablation task is triggered, the radio frequency ablation control apparatus 10 controls the radio frequency ablation catheter 60 to output the radio frequency energy to the ablation site by means of unipolar discharge, so as to execute an ablation operation on the ablation site.

Meanwhile, the controller 21 of the syringe pump 20 controls the syringe pump 20 to execute a perfusion operation on the ablation object, perfusing physiological saline into the ablation site, and acquiring the impedance value and/or temperature value of the ablation site in real time through the temperature acquisition device 30 and the impedance acquisition device 40. The controller 21 obtains real-time change information of the impedance value and/or temperature value by analyzing the acquired impedance value and/or temperature value, and then instructs the driving device 24 to drive the push rod 23 to move along the desired direction with the desired speed according to the real-time change information obtained by the analysis, so as to adjust the perfusion amount of the syringe pump 20 dynamically.

Referring to FIG. 4, which is a flowchart of a perfusion control method for syringe pump provided by an embodiment of the present application. The method may be implemented by the syringe pump 20 in FIG. 1, or may be implemented by the radio frequency ablation control apparatus 10 in FIG. 1, or may be implemented by other computer devices electrically coupled to the syringe pump. As shown in FIG. 4, the method particularly includes:
Step S401, when an ablation task is triggered, controlling the syringe pump to execute a perfusion operation on the ablation object, and acquiring the impedance value and/or temperature value of the ablation object in real time.

Particularly, the ablation task may be triggered when, for example, a preset trigger time is reached, a trigger instruction of the radio frequency ablation apparatus is received, or a notification event that a user performs an operation for triggering the ablation task is detected. Among them, the operation for triggering the ablation task is, for example, to press a physical or virtual button for triggering the ablation task.

Optionally, after each start of the syringe pump, a perfusion parameter is set to a preset initial value. The perfusion parameter may include but is not limited to: a perfusion flow rate, a total perfusion amount, a perfusion time, and the like.

When the ablation task is triggered, the syringe pump executes the perfusion operation on the ablation object according to the above-mentioned initial value. Meanwhile, at least one of the impedance value and the temperature value of the ablation object is acquired in real time by the temperature acquisition device and the impedance acquisition device, taking as reference data for dynamical adjustment of the perfusion amount of the syringe pump. The impedance value and the temperature value of the ablation object may be, for example, the impedance value and the temperature value of the ablation site. The impedance value may be a resistance value, for example.

Optionally, the impedance value and/or temperature value of the ablation object may further be acquired in real time as follows:
Firstly, acquiring an impedance sample value and/or a temperature sample value of the ablation object in real time, and filtering the acquired impedance sample value and/or temperature sample value; and then, judging whether the filtered impedance sample value and/or temperature sample value exceed/exceeds a preset warning value range. On the one hand, if the filtered impedance sample value and/or temperature sample value exceed/exceeds the preset warning value range, alarm information is output to remind the user that the operation is abnormal and whether the ablation operation needs to be stopped. On the other hand, if the filtered impedance sample value and/or temperature sample value do/does not exceed the preset warning value range, the minimum value or an average value of the filtered impedance sample value and/or temperature sample value within a preset period (for example, within 10 seconds) is taken as the acquired impedance value and/or temperature value in real time.

Step S402, analyzing the acquired impedance value and/or temperature value to obtain real-time change information of the impedance value and/or temperature value.

Particularly, at least one of the impedance value and the temperature value is analyzed to obtain the real-time change information of at least one of the impedance value and the temperature value. Optionally, the real-time change information may include but is not limited to a real-time change trend and a real-time change amplitude, wherein the real-time change trend is a change direction, for example, increasing or decreasing of the value, and the real-time change amplitude is a change degree, which may be an absolute value or a ratio, for example, an increased value, or an increased percentage.

It should be noted that the real-time change trend and the real-time change amplitude mentioned above refer to an overall change trend and an overall change amplitude.

Step S403, adjusting the perfusion amount of the syringe pump dynamically according to the real-time change information obtained from the analysis.

Particularly, the perfusion amount of the syringe pump is dynamically adjusted according to an analysis result from the step S402. If obtained from the analysis is the real-time change information of the temperature value, the perfusion amount of the syringe pump is dynamically adjusted according to the real-time change information of the temperature value. If obtained from the analysis is the real-time change information of the impedance value, the perfusion amount of the syringe pump is dynamically adjusted according to the real-time change information of the impedance value. If obtained from the analysis is the real-time change information of the temperature value and the real-time change information of the impedance value, the perfusion amount of the syringe pump is dynamically adjusted according to the real-time change information of the temperature value and the real-time change information of the impedance value at the same time.

It should be understood that the impedance of a human body is the total impedance containing resistance and capacitance of skin, blood, muscle, cell tissues and their joints of the human body, and is mainly determined by the resistance of the human body. When the human body is brought into contact with a charged body, the human body is regarded as a circuit element to be connected into the loop. In a process of cancer cell ablation, in addition to the temperature, the impedance of the human body will change therewith due to the influence of the radio frequency energy. Therefore, taking the impedance and/or temperature as a reference for adjusting the perfusion amount may be more accurate to determine the impact of the radio frequency energy on the human body, thereby making the volume of the liquid perfused into the ablation site more accurate and more in line with ablation requirements.

Optionally, the step S403 may be particularly implemented by the following steps:
S11, judging whether the temperature value presents a rising trend or a falling trend according to the real-time change information obtained from the analysis;
S12, controlling the syringe pump to increase a perfusion flow rate according to a preset increase and a trend change of the impedance value or temperature value when the temperature value presents the rising trend;
S13, controlling the syringe pump to decrease the perfusion flow rate according to a preset decrease and the trend change of the impedance value or temperature value when the temperature value presents the falling trend.

Optionally, the step S403 may further be particularly implemented as follows:
S21, judging whether the impedance value presents a rising trend or a falling trend according to the real-time change information obtained from the analysis;
S22, controlling the syringe pump to increase the perfusion flow rate according to a preset increase and a trend change of the impedance value or temperature value when the impedance value presents the rising trend;
S23, controlling the syringe pump to decrease the perfusion flow rate according to a preset decrease and the trend change of the impedance value or temperature value when the impedance value presents the falling trend.

Optionally, the step S403 may further be particularly implemented by the following steps:
S31, analyzing whether the real-time change trend and the real-time change amplitude of the impedance value and the real-time change trend and the real-time change amplitude of the temperature value meet corresponding adjustment conditions, respectively;
S32, when the real-time change trend and the real-time change amplitude of the impedance value and the real-time change trend and the real-time change amplitude of the temperature value meet their corresponding adjustment conditions, respectively, adjusting the perfusion amount of the syringe pump dynamically according to the real-time change trend and the real-time change amplitude of the impedance value;
S33, otherwise, when the real-time change trend and the real-time change amplitude of only one parameter of two parameters (i.e., the impedance value and the temperature value) meet the corresponding adjustment conditions, adjusting the perfusion amount of the syringe pump dynamically according to the real-time change trend and the real-time change amplitude of the parameter that meets the corresponding adjustment conditions.

The adjustment conditions mentioned above are conditions respectively met by the real-time change trend and the real-time change amplitude of the impedance value and the temperature value when the perfusion amount needs to be adjusted, for example, whether the real-time change trend and the real-time change amplitude of the impedance value and the temperature value present the rising trend or the falling trend, whether an increase reaches a preset amplitude and the like.

For the specific adjusting manner of the perfusion amount in the steps S31 to S33, reference may be made to the relevant description of dynamical adjustment of the perfusion amount of the syringe pump according to the real-time change information of the temperature value or the impedance value in the foregoing and following embodiments, which will be omitted here.

Optionally, the step S403 may further be particularly implemented by the following steps:
Determining a current ablation stage according to the real-time change trend and the real-time change amplitude of the impedance value, and then adjusting the perfusion amount of the syringe pump according to a target adjustment logic corresponding to the current ablation stage.

That is, the whole ablation process is divided into multiple ablation stages according to the real-time change trend and the real-time change amplitude of the impedance value, and then the perfusion amount of the syringe pump is adjusted according to the corresponding adjustment logic based on a change characteristic of the impedance value of each ablation stage, so as to make the adjustment more targeted, thereby further improving the accuracy of the perfusion.

Further, the step of determining the current ablation stage according to the real-time change trend and the real-time change amplitude of the impedance value and then adjusting the perfusion amount of the syringe pump according to the target adjustment logic corresponding to the current ablation stage may particularly include the following steps:
S41, when the syringe pump starts to perfuse a liquid into the ablation object, determining the beginning of a first ablation stage, and adjusting the perfusion amount of the syringe pump according to a first target adjustment logic based on the real-time change trend and the real-time change amplitude of the impedance value;
S42, when the impedance value presents a first change trend and reaches a first change amplitude in the first ablation stage, determining the beginning of a second ablation stage, and adjusting the perfusion amount of the syringe pump according to a second target adjustment logic;
S43, when the impedance value presents a second change trend and reaches a second change amplitude in the second ablation stage, determining the beginning of a third ablation stag, and adjusting the perfusion amount of the syringe pump according to a third target adjustment logic;
S44, when the impedance value presents a third change trend and reaches a third change amplitude in the third ablation stage, determining the beginning of a fourth ablation stage, and adjusting the perfusion amount of the syringe pump according to a fourth target adjustment logic.

Optionally, in another embodiment of the present application, during the process of executing the entire process of the perfusion operation, when the detected temperature value is greater than a first abnormal value or less than a second abnormal value for more than a first preset duration, the syringe pump is controlled to stop the perfusion operation and the ablation task is suspended, wherein the first preset duration is greater than or equal to zero. When the first preset duration is equal to 0, that is, as long as the detected temperature value is greater than the first abnormal value or less than the second abnormal value, the syringe pump is immediately controlled to stop the perfusion operation.

Optionally, in another embodiment of the present application, during the process of executing the entire process of performing the perfusion operation, when the detected impedance value is greater than a third abnormal value or less than a fourth abnormal value for more than a second preset duration, the syringe pump is controlled to stop the perfusion operation and the ablation task is suspended, wherein the second preset duration is greater than or equal to zero. When the second preset duration is equal to 0, that is, as long as the detected impedance value is greater than the third abnormal value or less than the fourth abnormal value, the syringe pump is immediately controlled to stop the perfusion operation.

In this way, when the detected temperature value or impedance value exceeds a preset range for more than the preset duration, the syringe pump is controlled to immediately stop the perfusion operation, and potential safety hazards may be discovered and eliminated in time, and thus the safety of the ablation operation is further improved.

It should be noted that the step S402 and the step S403 in this embodiment are not limited by their sequence numbers, and may be executed synchronously in practical applications.

Optionally, in another embodiment of the present application, when acquired data in real time are the impedance value and the temperature value of the ablation object, all adjustment amplitudes such as the increasing amplitude and the decreasing amplitude in the present application may be determined according to the change information of the impedance value and the temperature value. In this way, the adjustment amplitude is determined in combination with the change of the impedance value and the change of the temperature value, which may further improve the accuracy of adjustment of the perfusion amount.

Particularly, different base values and weight values are assigned to the real-time change amplitude of the impedance value and the real-time change amplitude of the temperature value. During the process of dynamical adjustment of the perfusion amount of the syringe pump, the adjustment amplitude is calculated according to the base value and the weight value, wherein the weight value corresponding to the real-time change amplitude of the temperature value is smaller than the weight value corresponding to the real-time change amplitude of the impedance value. Calculating the adjustment amplitude according to the base value and the weight value may particularly employ a weighted average manner or a weighted manner.

In the embodiment of the present application, when the ablation task is triggered, the syringe pump is controlled to execute the perfusion operation on the ablation object, and the impedance value and/or temperature value of the ablation object are acquired in real time; and then, the acquired impedance value and/or temperature value are/is analyzed, and the perfusion amount of the syringe pump is dynamically adjusted according to the real-time change information of the impedance value and/or temperature value obtained from the analysis. Accordingly, automatic, dynamic adjustment of the perfusion amount of the syringe pump is realized based on the analysis of changes of the impedance value and/or temperature value of the ablation object during the process of executing the ablation task. Since the perfusion amount of the syringe pump is adjusted automatically and dynamically along with the changes of the impedance value and/or temperature value of the ablation object, operation delays and errors caused by manual determination can be reduced, and the timeliness and the accuracy of liquid perfusion during the process of executing the ablation task can be improved. Accordingly, the injury of the ablation operation to the ablation object is reduced, and the safety of the radio frequency ablation treatment is improved.

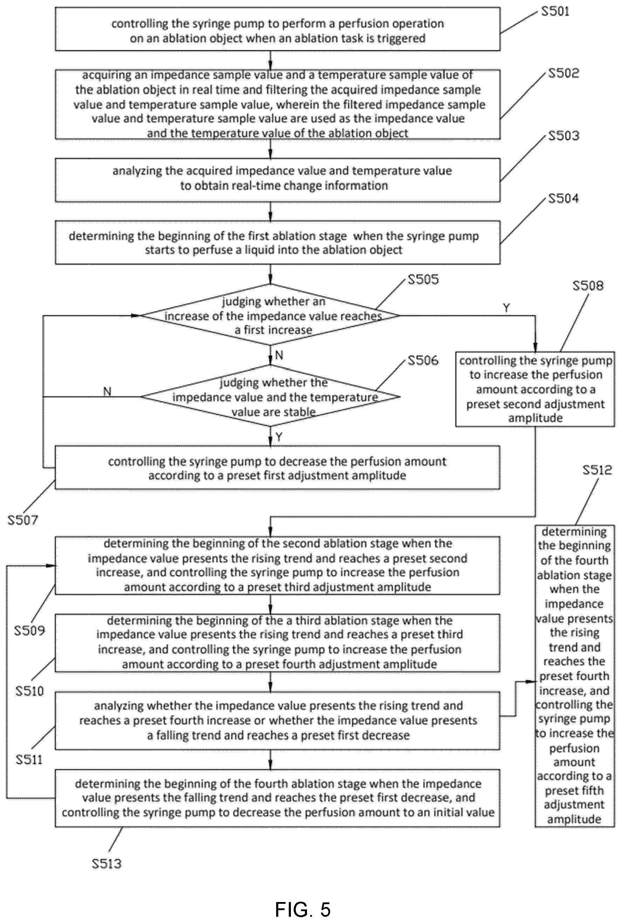

Referring to FIG. 5, which is a flowchart of a perfusion control method for syringe pump according to another embodiment of the present application. The method may be implemented by the syringe pump 20 in FIG. 1, or may be implemented by the radio frequency ablation control apparatus 10 in FIG. 1, or may be implemented by other computer devices electrically coupled to the syringe pump. As shown in FIG. 5, the method particularly includes the following steps:
Step S501, controlling the syringe pump to perform a perfusion operation on an ablation object when an ablation task is triggered.
Step S502, acquiring an impedance sample value and a temperature sample value of the ablation object in real time and filtering the acquired impedance sample value and temperature sample value, wherein the filtered impedance sample value and temperature sample value are used as the impedance value and the temperature value of the ablation object.

Particularly, the ablation task may be triggered when, for example, a preset trigger time is reached, a trigger instruction of the radio frequency ablation apparatus is received, or a notification event that a user performs an operation for triggering the ablation task is detected. Among them, the operation for triggering the ablation task is, for example, to press a physical or virtual button for triggering the ablation task.

Optionally, after each start of the syringe pump, a perfusion parameter is set to a preset initial value. The perfusion parameter may include but is not limited to a perfusion flow rate, a total perfusion amount, a perfusion time, and the like.

When the ablation task is triggered, the syringe pump executes the perfusion operation on the ablation object according to the initial value mentioned above. Meanwhile, the impedance sample value and the temperature sample value of the ablation object are acquired in real time by the temperature acquisition device and the impedance acquisition device, and the acquired impedance sample value and temperature sample value are filtered to remove abnormal values in the samples. Then, the filtered impedance sample value and temperature sample value are used as the impedance value and temperature value of the ablation object.

It should be understood that filtering is to process noise and invalid values in the acquired data due to the interference of a sample circuit and an error code during transmitting of the samples, so as to reduce an acquisition error to a preset range.

In this way, the referenceability of the acquired data may be further improved by filtering the abnormal values in the samples.

Optionally, in another embodiment of the present application, after the impedance sample value and the temperature sample value of the ablation object are acquired in real time and the acquired impedance sample value and temperature sample value are filtered, further includes judging whether the filtered impedance sample value and temperature sample value exceed a preset warning value range. When the filtered impedance sample value and temperature sample value exceed the preset warning value range, alarm information will be output to remind the user that the operation is abnormal and whether the ablation operation needs to be stopped. When the filtered impedance sample value and temperature sample value do not exceed the preset warning value range, the minimum value or an average value of the filtered impedance sample value and temperature sample value within a preset period is taken as the acquired impedance value and temperature value in real time, for example, the minimum value of the filtered impedance sample value within 10s is taken as the acquired impedance value in real time, and the minimum value of the filtered temperature sample value within 10s is taken as the acquired temperature value in real time.

In this way, the safety of the ablation operation may be further improved by alarming when the detected filtered impedance sample value and temperature sample value exceed the preset warning value range. In addition, the referenceability of the acquired data may be further improved by using the minimum value or average value within the preset period as the acquired impedance value and the temperature value in real time.

Step S503, analyzing the acquired impedance value and temperature value to obtain real-time change information of the impedance value and the temperature value.

Particularly, the real-time change information may include but is not limited to a real-time change trend and a real-time change amplitude. The real-time change trend is a change direction, for example, the increasing or decreasing of the value. The real-time change amplitude is a change degree, which may be an absolute value or a ratio, for example, an increased value, or an increased percentage.

Step S504, when the syringe pump starts to perfuse a liquid into the ablation object, determining the beginning of the first ablation stage.

Step S505, according to the real-time change information of the impedance value, judging whether an increase of the impedance value reaches a first increase:

When the increase of the impedance value does not reach the first increase, it performs step S506: judging whether the impedance value and the temperature value are stable according to the real-time change information of the impedance value and the temperature value:
When the impedance value and the temperature value are stable, it performs step S507: controlling the syringe pump to decrease the perfusion amount according to a preset first adjustment amplitude, and returning to step S505.

When the impedance value and the temperature value are not stable, it performs step S505.

When the increase of the impedance value reaches the first increase, it performs step S508: controlling the syringe pump to increase the perfusion amount according to a preset second adjustment amplitude.

Particularly, judging whether the acquired impedance value of the ablation object in real-time presents a rising trend and reaches a preset first increase may be, for example, judge whether the acquired obtained impedance value in real-time increases 1%. It should be understood that the increase involved in the present application represents a rising degree, which may be an absolute value or a proportional value. The trend involved in the present application may be an overall trend. In practical applications, acquiring impedance values and temperature values in real time, analyzing and judging the real-time change information, and adjusting the perfusion amount may be performed in parallel by multithreading.

On the one hand, if the impedance value presents an overall rising trend but does not reach the preset first increase, judge whether the impedance value and the temperature value are stable according to the real-time change information of the impedance value and the temperature value, that is, judge whether the impedance value and the temperature value are respectively within or are maintained within their respective corresponding preset impedance value ranges and preset temperature value ranges. If the impedance value and the temperature value are respectively within or are maintained within their respective corresponding preset impedance value ranges and preset temperature value ranges, then it is determined that the impedance value and the temperature value are stable; and otherwise, it is determined that the impedance value and the temperature value are not stable.

If the impedance value and the temperature value are stable, it means that the current perfusion amount is slightly large and the increase or rising speed of the impedance value and the temperature value does not meet the specified requirements, the syringe pump is thus controlled to decrease the perfusion volume according to the preset first adjustment amplitude in order to avoid unnecessary waste of the perfused liquid and over liquid perfusion, and it returns to perform step S505 to judge whether the increase of the impedance value reaches the first increase according to the real-time change information of the impedance value.

If the impedance value and the temperature value are not stable, it means that the current perfusion amount is moderate and adjustment is not needed, it returns to perform step S505 to judge whether the increase of the impedance value reaches the first increase according to the real-time change information of the impedance value.

It should be understood that during the process of executing the ablation operation, the impedance value of the ablation site changes along with the increase of the radio frequency energy (heat), and presents a rising trend as a whole. As the temperature of the ablation site increases, protein is denatured and carbonized, and its inherent physical impedance characteristics will change therewith. That is, as the ablation operation is performed continuously, the impedance value and the temperature value of the ablation site will change therewith, thereby presenting an unstable state. The purposes of perfusing physiological saline are to lower the temperature so as to avoid injury to the patient due to excessive temperature and to reduce the impedance such that an ablation power is in a normal range. As the ablation operation is performed continuously, it is necessary to make the impedance value and the temperature value of the ablation site change periodically as required for ablation. For example, in the first and second ablation stages, it is necessary to keep the impedance value and the temperature value rise as a whole. If the actual impedance value and the actual temperature value are in a stable state, i.e., maintained within a certain range, the perfusion amount is too large to meet the requirement for the current ablation.

On the other hand, if the impedance value presents a rising trend and reaches the preset first increase, the syringe pump is controlled to increase the perfusion amount according to the preset second adjustment amplitude. Particularly, the perfusion amount may be increased by increasing the perfusion flow rate of the liquid.

Step S509, when the impedance value presents the rising trend and reaches a preset second increase, determining the beginning of the second ablation stage, and controlling the syringe pump to increase the perfusion amount according to a preset third adjustment amplitude.

Particularly, in the first ablation stage, the real-time change trend and the real-time change amplitude of the impedance value are continuously analyzed, and when the impedance value present the rising trend and reaches the preset second increase (for example, increases increasing 2%), the second ablation stage begins and the syringe pump is controlled to increase the perfusion amount according to the preset third adjustment amplitude.

Step S510, when the impedance value presents the rising trend and reaches a preset third increase, determining the beginning of the a third ablation stage, and controlling the syringe pump to increase the perfusion amount according to a preset fourth adjustment amplitude.

Particularly, in the second ablation stage, the real-time change trend and the real-time change amplitude of the impedance value are continuously analyzed, and when the impedance value presents the rising trend and reaches the preset third increase (for example, increasing 3%), the third ablation stage begins and the syringe pump is controlled to increase the perfusion amount according to the preset fourth adjustment amplitude.

Step S511, analyzing whether the impedance value presents the rising trend and reaches a preset fourth increase or whether the impedance value presents a falling trend and reaches a preset first decrease:
Step S512, when the impedance value presents the rising trend and reaches the preset fourth increase, determining the beginning of the fourth ablation stage, and controlling the syringe pump to increase the perfusion amount according to a preset fifth adjustment amplitude.
Step S513, when the impedance value presents the falling trend and reaches the preset first decrease, determining the beginning of the fourth ablation stage, and controlling the syringe pump to decrease the perfusion amount to an initial value.

Particularly, in the third ablation stage, the real-time change trend and the real-time change amplitude of the impedance value are continuously analyzed. It should be noted that in the third ablation stage, in addition to analyzing whether the impedance value presents the rising trend, it is further necessary to analyze whether the impedance value presents the falling trend. That is, when the impedance value presents the rising trend and reaches the preset fourth increase (for example, increasing 4%) or the impedance value presents the falling trend and reaches the preset first decrease (for example, decreasing 4%), it is determined that the fourth ablation stage begins.

However, a processing logic varies according to different reasons for confirming the beginning of the fourth ablation stage. When the beginning of the fourth ablation stage is determined because the impedance value presents the rising trend and reaches the preset fourth increase, the syringe pump is controlled to increase the perfusion amount according to the preset fifth adjustment amplitude. When the beginning of the fourth ablation stage is determined because the impedance value presents the falling trend and reaches the preset first decrease, the syringe pump is controlled to decrease the perfusion amount to the initial value and the first ablation stage begins, and then preforms step S509: when the impedance value presents the rising trend and reaches the preset second increase, determining the beginning of the second ablation stage and controlling the syringe pump to increase the perfusion amount according to the preset third adjustment amplitude until this control period is ended.

It should be understood that above-mentioned controlling the syringe pump to decrease the perfusion amount to the initial value may be done at one time, or may be done step by step. The above-mentioned control period may refer to a completion period of the entire ablation task, or a single ablation task may be divided into multiple control periods as well.

It should be understood that values of the preset first increase to the preset fourth increase and the preset first decrease may be the same or different in specific applications. Similarly, values of the first adjustment amplitude to the fifth adjustment amplitude may be the same or different in specific applications. The specific values may be set and adjusted at any time according to a custom operation of a user in practical applications.

It should be noted that the judging in the embodiments of the present application are all based on the real-time change information of the corresponding parameters.

In the embodiment of the present application, the entire ablation process is divided into multiple ablation stages according to the real-time change trend and the real-time change amplitude of the impedance value of the ablation object, and the perfusion amount of the syringe pump is dynamically adjusted according to a corresponding adjustment logic based on change characteristics of the impedance value and the temperature value of the ablation object acquired in real time in each ablation stage, which makes the adjustment more targeted, thereby improving the accuracy of automatic perfusion control.

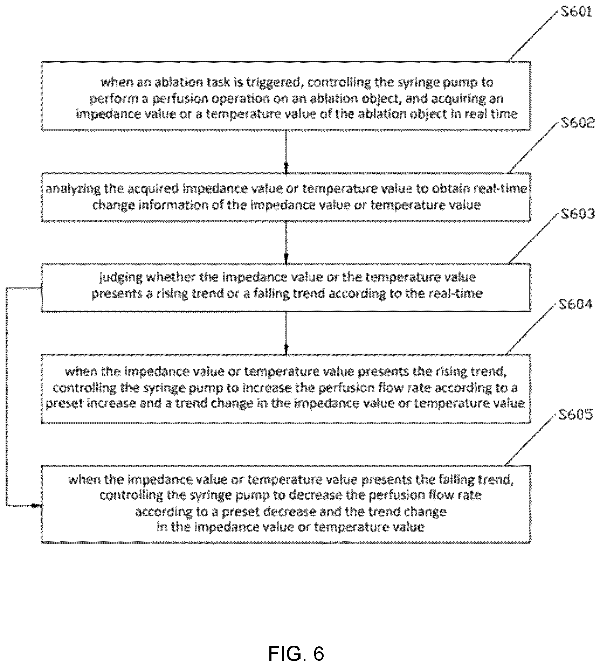

Referring to FIG. 6, which is a flow chart of a perfusion control method for syringe pump according to another embodiment of the present application. The method may be implemented by the syringe pump 20 in FIG. 1, or may be implemented by the radio frequency ablation control apparatus 10 in FIG. 1, or may be implemented by other computer devices electrically coupled to the syringe pump. As shown in FIG. 6, the method particularly includes the following steps:
Step S601, when an ablation task is triggered, controlling the syringe pump to perform a perfusion operation on an ablation object, and acquiring an impedance value or a temperature value of the ablation object in real time.

Particularly, the ablation task may be triggered when, for example, a preset trigger time is reached, a trigger instruction of the radio frequency ablation apparatus is received, or a notification event that a user performs an operation for triggering the ablation task is detected. Among them, the operation for triggering the ablation task is for example to press a physical or virtual button for triggering the ablation task.

Optionally, after each start of the syringe pump, a perfusion parameter is set to a preset initial value. The perfusion parameter may include but is not limited to a perfusion flow rate, a total perfusion amount, a perfusion time, and the like.

When the ablation task is triggered, the syringe pump performs the perfusion operation on the ablation object according to the above-mentioned initial value. Meanwhile, the impedance value or the temperature value of the ablation object is acquired in real time by the temperature acquisition device and the impedance acquisition device, taking as reference data for dynamical adjustment of the perfusion amount of the syringe pump. The impedance value and the temperature value of the ablation object may be for example the impedance value and the temperature value of the ablation site. The impedance value may be a resistance value, for example.

Optionally, the impedance value or the temperature value of the ablation object may further be acquired in real time particularly by the following steps:
acquiring an impedance sample value or a temperature sample value of the ablation object in real time, and filtering the acquired impedance sample value or temperature sample value;
judging whether the filtered impedance sample value or temperature sample value exceeds a preset warning value range:
when the filtered impedance sample value or temperature sample value exceeds the preset warning value range, outputting alarm information to remind the user that the operation is abnormal and whether the ablation operation needs to be stopped;
when the filtered impedance sample value or temperature sample value does not exceed the preset warning value range, taking the minimum value or an average value of the filtered impedance sample value and temperature sample value within a preset period (for example, within 10s) as the acquired impedance value or temperature value in real time.

Step S602, analyzing the acquired impedance value or temperature value to obtain real-time change information of the impedance value or temperature value.

Particularly, the real-time change information may include but is not limited to a real-time change trend and a real-time change amplitude. The real-time change trend is a change direction, for example, the increasing or decreasing of the value. The real-time change amplitude is a change degree, which may be an absolute value or a proportional value, for example, an increased value, or an increased percentage.

Optionally, in another embodiment of the present application, the impedance value and the temperature value of the ablation object may be acquired in real time simultaneously, and the acquired impedance value and temperature value may be analyzed. At this time, all the adjustment amplitudes such as the increase and the decrease involved in the following may be determined according to analysis results.

Particularly, different base values and weight values are assigned to the real-time change amplitude of the impedance value and the real-time change amplitude of the temperature value. During the process of dynamical adjustment of the perfusion amount of the syringe pump, the adjustment amplitude is calculated according to the base value and the weight value, wherein the weight value corresponding to the real-time change amplitude of the temperature value is smaller than the weight value corresponding to the real-time change amplitude of the impedance value. Calculating the adjustment amplitude according to the base value and the weight value may particularly employ a weighted average manner or a weighted manner.

Step S603, judging whether the impedance value or the temperature value presents a rising trend or a falling trend according to the real-time change information obtained from the analysis.

Step S604, when the impedance value or the temperature value presents the rising trend, controlling the syringe pump to increase the perfusion flow rate according to a preset increase and a trend change in the impedance value or the temperature value.

Step S605, when the impedance value or the temperature value presents the falling trend, controlling the syringe pump to decrease the perfusion flow rate according to a preset decrease and the trend change in the impedance value or the temperature value.

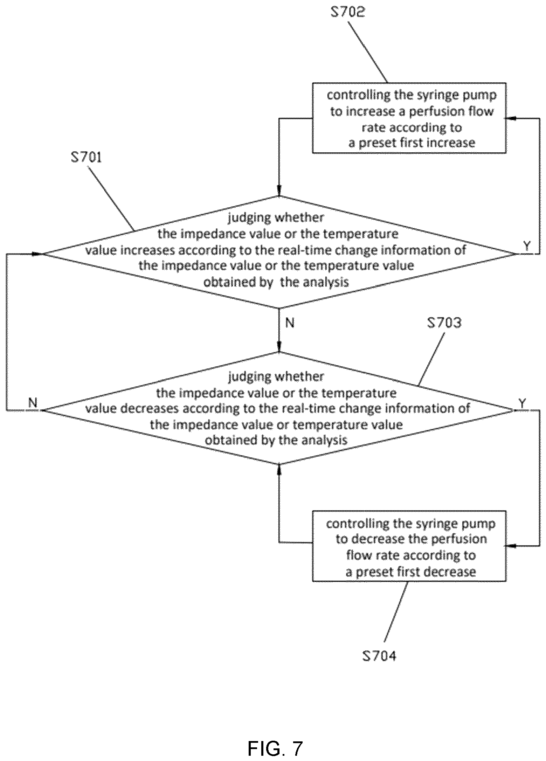

Particularly, as shown in FIG. 7, the steps S603 to S605 may be implemented through the following steps:
Step S701, judging whether the impedance value or the temperature value increases according to the real-time change information of the impedance value or the temperature value obtained from the analysis:
When the impedance value or the temperature value increases, it performs step S702: controlling the syringe pump to increase a perfusion flow rate according to a preset first increase so as to increase the perfusion amount, and then returning to perform step S701.

When the impedance value or the temperature value does not increase, it performs step S703: judging whether the impedance value or the temperature value decreases according to the real-time change information of the impedance value or the temperature value obtained from the analysis:
When the impedance value or the temperature value decreases, it performs step S704: controlling the syringe pump to decrease the perfusion flow rate according to a preset first decrease so as to decrease the perfusion amount, and returning to perform step S703.

When the impedance value or the temperature value does not decrease, it returns to perform step S701. This cycle repeats until the control period is ended.

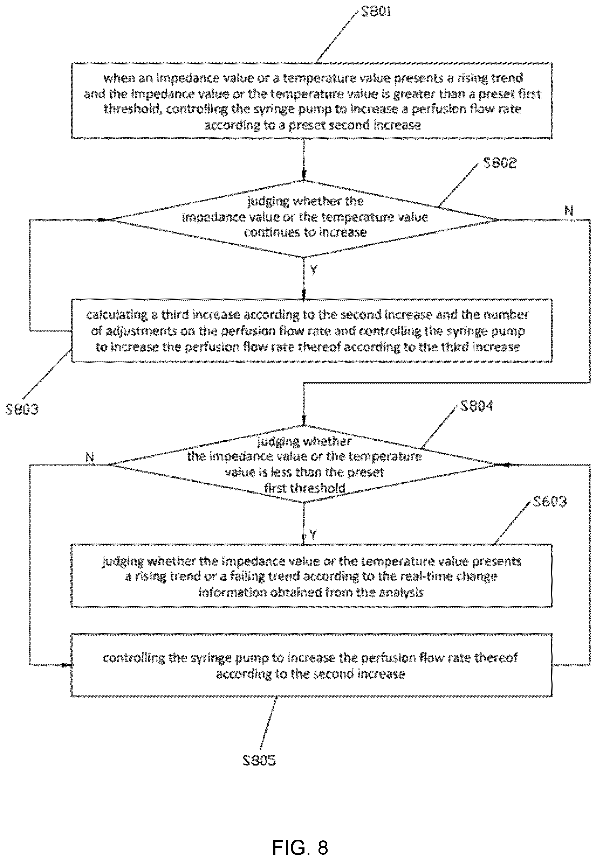

Optionally, in another embodiment of the present application, as shown in FIG. 8, the step S604 may be further implemented by the following steps:
Step S801, when an impedance value or a temperature value presents a rising trend and the impedance value or the temperature value is greater than a preset first threshold, controlling the syringe pump to increase a perfusion flow rate according to a preset second increase.
Step 802, judging whether the impedance value or the temperature value continues to increase:
   If the impedance value or the temperature value continues to increase, it performs step S803: calculating a third increase according to the second increase and the number of adjustments on the perfusion flow rate and controlling the syringe pump to increase the perfusion flow rate thereof according to the third increase, and returns to perform step S802 to judge whether the impedance value or the temperature value continues to increase until the perfusion flow rate increases to a preset maximum flow rate or until the impedance value or temperature value is less than the preset first threshold.

If the impedance value or the temperature value does not continue to increase, it performs step S804: judging whether the impedance value or the temperature value is less than the preset first threshold.

If the impedance value or the temperature value is not less than the preset first threshold, it performs step S805: controlling the syringe pump to increase the perfusion flow rate thereof according to the second increase until the perfusion flow rate increases to the preset maximum flow rate or until the impedance value or temperature value is less than the preset first threshold.

If the impedance value or the temperature value is less than the preset first threshold, it returns to perform step S603 to judge whether the impedance value or the temperature value presents the rising trend or the falling trend according to the real-time change information obtained from the analysis.

This cycle repeats until the control period is ended.

Optionally, calculating the third increase according to the second increase and the number of the adjustments on the perfusion flow rate in the step S803 may particularly be using a value obtained through multiplying the second increase by the number of the adjustments as the third increase; or the third increase may be calculated according to a geometric incremental rule based on a preset ratio, the second increase, and the number of the adjustments on the perfusion flow rate. The number of the adjustments on the perfusion flow rate may be understood as the number of operations performed to judge whether the impedance value or the temperature value continues to increase.

Further, when the perfusion flow rate increases to the preset maximum flow rate, reminding information is output to remind the user that the perfusion flow rate of the syringe pump has reached a limit value, wherein the reminding information may be output in at least one of speech, text, image, animation and light.

It should be understood that the purpose of adjusting the perfusion amount is to keep the impedance value and the temperature value of the ablation object stable within a safe range. On the one hand, if the impedance value or the temperature value does not continue to increase, it is effective to increase the perfusion flow rate of the syringe pump according to the previous increase. At this time, the perfusion flow rate of the syringe pump is continuously increased according to the increase, which may avoid excessive adjustment. As a result, the accuracy of the perfusion may be improved.

On the other hand, if the impedance value or the temperature value continues to increase, the effect of increasing the perfusion flow rate of the syringe pump according to the previous increase is poor, and the increase may not meet the ablation demand. At this time, the syringe pump is controlled to increase the perfusion flow rate thereof according to a larger increase, such that the perfusion amount is quickly adjusted to a desired degree. Accordingly, the timeliness of the perfusion is improved.

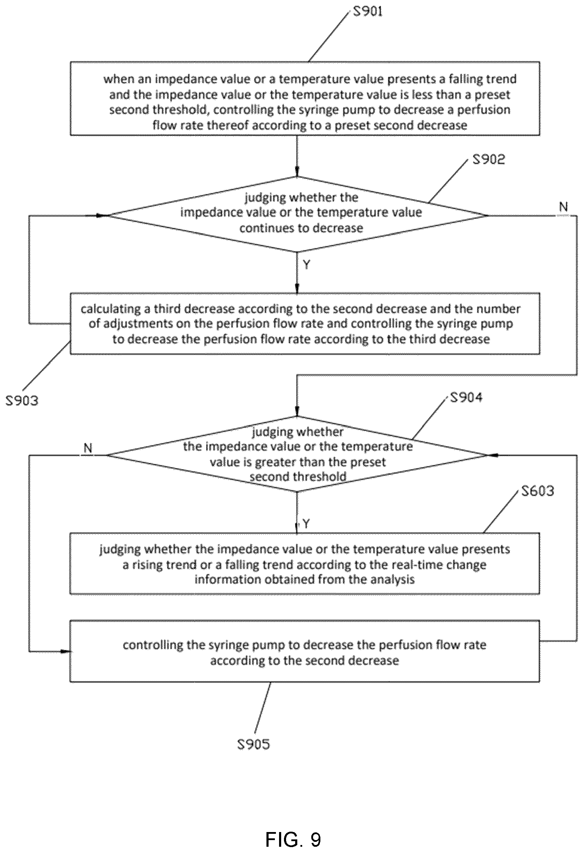

Optionally, in another embodiment of the present application, as shown in FIG. 9, the step S605 may be implemented by the following steps:
Step S901, when an impedance value or a temperature value presents a falling trend and the impedance value or the temperature value is less than a preset second threshold, controlling the syringe pump to decrease a perfusion flow rate thereof according to a preset second decrease.
Step S902, judging whether the impedance value or the temperature value continues to decrease.

If the impedance value or the temperature value continues to decrease, it performs step S903: calculating a third decrease according to the second decrease and the number of adjustments on the perfusion flow rate and controlling the syringe pump to decrease the perfusion flow rate according to the third decrease, and returns to perform step S902 to judge whether the impedance value or the temperature value continues to decrease until the perfusion flow rate decreases to a preset minimum flow rate or until the impedance value or the temperature value is greater than the preset second threshold.

If the impedance value or the temperature value does not continue to decrease, it performs step S904: judging whether the impedance value or the temperature value is greater than the preset second threshold.

If the impedance value or the temperature value is not greater than the preset second threshold, it performs step S905: controlling the syringe pump to decrease the perfusion flow rate according to the second decrease, and returns to perform step S904 to judge whether the temperature value or the temperature value is greater than the preset second threshold until the perfusion flow rate decreases to the preset minimum flow rate or until the impedance value or the temperature value is greater than the preset second threshold.

If the impedance value or the temperature value is greater than the preset second threshold, it returns to perform step S603 to judge whether the impedance value or the temperature value presents the rising trend or the falling trend according to the real-time change information obtained from the analysis.

It should be understood that if the impedance value or the temperature value does not continue to decrease, it is effective to decrease the perfusion flow rate of the syringe pump according to the previous decrease. At this time, the perfusion flow rate of the syringe pump is continuously decreased according to the decrease, which may avoid excessive. As a result, the accuracy of the perfusion may be improved. On the other hand, if the impedance value or the temperature value continues to decrease, the previous decrease is not enough. At this time, the decrease is improved, such that the perfusion amount may be rapidly adjusted to a desired degree, and thus the timeliness of the perfusion is improved.

This cycle repeats until the control period is ended.

Optionally, calculating the third increase according to the second increase and the number of the adjustments on the perfusion flow rate in step S903 may particularly be using a value obtained through multiplying the second decrease by the number of the adjustments as the third decrease, or the third decrease may be calculated according to a geometric degressive rule based on a preset ratio, the second decrease, and the number of the adjustments on the perfusion flow rate. The number of the adjustments on the perfusion flow rate may be understood as the number of operations performed to judge whether the impedance value or the temperature value continues to decrease.

Further, when the perfusion flow rate decreases to the preset minimum flow rate, reminding information is output to remind the user that the perfusion flow rate of the syringe pump has been decreased to a limit value, wherein the reminding information may be output in at least one of speech, text, image, animation and light.

It should be understood that the above-mentioned preset first threshold is the preset maximum limit, and the above-mentioned preset second threshold is the preset minimum limit. It is possible to cause injury to the ablation object when the impedance value or temperature value exceeding these two thresholds, and thus it is necessary to adjust the perfusion amount. In addition, the preset first threshold and the preset second threshold are a collective term. In practical applications, specific values and units of the preset first threshold and the preset second threshold are determined by types of objects to which they are applied, and may be set according to user-defined operations. That is, the specific values and units of the preset first threshold and the preset second threshold applied to judge the impedance value are different from that applied to judge the temperature value. For example, when applied to judge the impedance value, the unit of the preset first threshold is ohm; but when applied to the judge the temperature value, the unit of the preset first threshold is degrees Celsius.

In addition, all the increase and decrease involved in the present application represent a degree, which may be an absolute value or a proportional value, such as an increased value or an increased percentage.

In the embodiment of the present application, when the ablation task is triggered, the syringe pump is controlled to perform the perfusion operation on the ablation object, and the impedance value and/or temperature value of the ablation object are acquired in real time; and then the acquired impedance value and/or temperature value are/is analyzed, and the perfusion amount of the syringe pump is dynamically adjusted according to real-time change information of the impedance value and/or temperature value obtained from the analysis. Accordingly, automatic dynamic adjustment of the perfusion amount of the syringe pump is realized based on the analysis of changes in the impedance value and/or temperature value of the ablation object during the process of executing the ablation task. Since the perfusion amount of the syringe pump is adjusted automatically and dynamically along with the changing of the impedance value and/or temperature value of the ablation object, operation delays and errors caused by manual determination can be reduced, and the timeliness and the accuracy of liquid perfusion during the process of executing the ablation task can be improved. Accordingly, the injury of the ablation operation to the ablation object is reduced, and the safety of the radio frequency ablation treatment is improved.

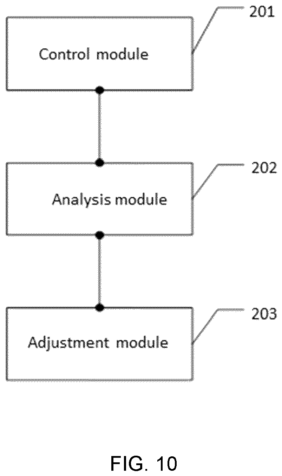

Referring to FIG. 10, which is a schematic diagram of a perfusion control apparatus for syringe pump provided by an embodiment of the present application. For facilitating description, only parts related to the embodiments of the present application are shown. The apparatus may be disposed in the syringe pump 20 shown in FIG. 1, or in the radio frequency ablation control apparatus 10, or may be disposed in other computer devices. The apparatus includes a control module 201, an analysis module 202 and an adjustment module 203.

The control module 201 is configured to control a syringe pump to perform a perfusion operation on an ablation object, and acquire an impedance value and/or a temperature value of the ablation object in real time when an ablation task is triggered.

The analysis module 202 is configured to analyze the acquired impedance value and/or temperature value to obtain real-time change information of the impedance value and/or temperature value.

The adjustment module 203 is configured to dynamically adjust a perfusion amount of the syringe pump according to the real-time change information obtained from the analysis.

Optionally, the real-time change information includes a real-time change trend and a real-time change amplitude.

Optionally, the adjustment module 203 is further configured to determine a current ablation stage according to the real-time change trend and the real-time change amplitude of the impedance value, and adjust the perfusion amount of the syringe pump according to a target adjustment logic corresponding to the current ablation stage.

Optionally, the adjustment module 203 includes:
a first adjustment module, which is configured to adjust a perfusion amount of the syringe pump according to a first target adjustment logic based on the real-time change trend and the real-time change amplitude of the impedance value when the syringe pump starts to perfuse a liquid into the ablation object, i.e., a first ablation stage begins;
a second adjustment module, which is configured to adjust the perfusion amount of the syringe pump according to a second target adjustment logic when the impedance value presents a first real-time change trend and reaches a first real-time change amplitude in the first ablation stage, i.e., a second ablation stage begins;
a third adjustment module, which is configured to adjust the perfusion amount of the syringe pump according to a third target adjustment logic when the impedance value presents a second real-time change trend and reaches a second real-time change amplitude in the second ablation stage, i.e., a third ablation stage begins; and
a fourth adjustment module, which is configured to adjust the perfusion amount of the syringe pump according to a fourth target adjustment logic when the impedance value presents a third real-time change trend and reaches a third real-time change amplitude in the third ablation stage, i.e., a fourth ablation stage begins.

Optionally, when the acquired data in real-time includes the impedance value and the temperature value, the first adjustment module is further configured to judge whether the impedance value presents a rising trend and reaches a preset first increase: if the impedance value presents the rising trend but does not reach the preset first increase, judging whether the impedance value and the temperature value are stable; if the impedance value and the temperature value are stable, controlling the syringe pump to decrease the perfusion amount according to the preset first adjustment amplitude and returning to perform the step of judging whether the impedance value presents the rising trend and reaches the preset first increase; and if the impedance value presents the rising trend and reaches the preset first increase, controlling the syringe pump to increase the perfusion amount according to a preset second adjustment amplitude.

Optionally, the second adjustment module is further configured to control the syringe pump to increase the perfusion amount according to a preset third adjustment amplitude when the impedance value presents the rising trend and reaches the preset second increase in the first ablation stage, which means the second ablation stage begins.

Optionally, the third adjustment module is further configured to control the syringe pump to increase the perfusion amount according to a preset fourth adjustment amplitude when the impedance value presents the rising trend and reaches the preset third increase in the second ablation stage, which means the third ablation stage begins.

Optionally, the fourth adjustment module is further configured to control the syringe pump to increase the perfusion amount according to a preset fifth adjustment amplitude when the impedance value presents the rising trend and reaches the preset fourth increase in the third ablation stage, i.e., the fourth ablation stage begins; or, control the syringe pump to decrease the perfusion amount to an initial value when the impedance value presents a falling trend and reaches a preset first decrease, i.e., the fourth ablation stage begins.

Optionally, the adjustment module 203 is further configured to analyze whether the impedance value or the temperature value presents the rising trend or the falling trend: if the impedance value or the temperature value presents the rising trend, controlling the syringe pump to increase a perfusion flow rate according to a preset increase and a trend change in the impedance value or the temperature value; and if the impedance value or the temperature value presents the falling trend, controlling the syringe pump to decrease the perfusion flow rate according to a preset decrease and the trend change in the impedance value or the temperature value.

Optionally, the adjustment module 203 is further configured to judge whether the impedance value or the temperature value increases according to the real-time change information: if the impedance value or the temperature value increases, controlling the syringe pump to increase the perfusion flow rate according to the preset first increase and returning to perform the step of judging whether the impedance value or the impedance value decreases according to the real-time change information; if the impedance value or the temperature value does not increase, judging whether the impedance value or the temperature value decreases according to the real-time change information; if the impedance value or the temperature value decreases, controlling the syringe pump to decrease the perfusion flow rate according to the preset first decrease and returning to the perform step of judging whether the impedance value or the temperature value decreases according to the real-time change information; and if the impedance value or the temperature value does not decrease, returning to perform the step of judging whether the impedance value or the temperature value increases according to the real-time change information.

Optionally, the adjustment module 203 is further configured to: control the syringe pump to increase the perfusion flow rate according to a preset second increase when the impedance value or the temperature value is greater than a preset first threshold; judge whether the impedance value or the temperature value continues to increase; if the impedance value or the temperature value continues to increase, it calculates a third increase according to the second increase and the number of adjustments on the perfusion flow rate; control the syringe pump to increase the perfusion flow rate according to the third increase and return to perform the step of judging whether the impedance value or the temperature value continues to increase until the perfusion flow rate increases to a preset maximum flow rate; if the impedance value or the temperature value does not increase continuously, it judges whether the impedance value or the temperature value is less than the preset first threshold; if the impedance value or the temperature value is not less than the preset first threshold, it controls the syringe pump to increase the perfusion flow rate according to the second increase until the perfusion flow rate increases to the preset maximum flow rate; and if the impedance value or the temperature value is less than the preset first threshold, it returns to perform the step of judging whether the impedance value or the temperature value presents the rising trend or the falling trend according to the real-time change information obtained from the analysis.

Optionally, the adjustment module 203 is further configured to: control the syringe pump to decrease the perfusion flow rate of the syringe pump according to a preset second decrease when the impedance value or the temperature value is less than a preset second threshold,; judge whether the impedance value or the temperature value continues to decrease; if the impedance value or the temperature value continues to decrease, it calculates a third decrease according to the second decrease and the number of adjustments on the perfusion flow rate; control the syringe pump to decrease the perfusion flow rate according to the third decrease and return to perform the step of judging whether the impedance value or the temperature value continues to decrease until the perfusion flow rate decreases a preset minimum flow rate; if the impedance value or the temperature value does not decrease continuously, it judges whether the impedance value or the temperature value is greater than the preset second threshold; if the impedance value or the temperature value is not greater than the preset second threshold, it controls the syringe pump to decrease the perfusion flow rate according to the second decrease and returns to perform the step of judging whether the temperature value or the temperature value is greater than the preset second threshold until the perfusion flow rate decreases to the preset minimum flow rate; and if the impedance value or the temperature value is greater than the preset second threshold, it returns to perform the step of judging whether the impedance value or the temperature value presents the rising trend or the falling trend according to the real-time change information obtained from the analysis.

Optionally, the control module 201 includes:
a first data acquiring module, which is configured to acquire an impedance sample value and/or a temperature sample value of the ablation object in real time; and
a first filtering module, which is configured to filter the acquired impedance sample value and/or temperature sample value, wherein the filtered impedance sample value and/or temperature sample value are/is taken as the impedance value and/or temperature value.

Optionally, the control module 202 further includes:
a second data acquiring module, which is configured to acquire an impedance sample value and/or a temperature sample value of the ablation object in real time;
a second filtering module, which is configured to filter the acquired impedance sample value and/or temperature sample value;
a judging module, which is configured to judge whether the filtered impedance sample value and/or temperature sample value exceed/exceeds a preset warning value range;
an alarm module, which is configured to output alarm information when the filtered impedance sample value and/or temperature sample value exceed/exceeds the preset warning value range;
wherein the second data acquiring module is further configured to take the minimum value or an average value of the filtered impedance sample value and/or temperature sample value within a preset period as the impedance value and/or temperature value when the filtered impedance sample value and/or temperature sample value do/does not exceed the preset warning value range.

Optionally, the adjustment module 203 further includes:
a fifth adjustment module, which is configured to analyze whether the real-time change trend and the real-time change amplitude of the impedance value and the real-time change trend and the real-time change amplitude of the temperature value meet an adjustment condition when the real-time change information includes the real-time change trend and the real-time change amplitude of the impedance value and the real-time change trend and the real-time change amplitude of the temperature value; and when the real-time change trend and the real-time change amplitude of the impedance value and the real-time change trend and the real-time change amplitude of the temperature value meet the adjustment condition, adjust the perfusion amount of the syringe pump dynamically according to the real-time change trend and the real-time change amplitude of the impedance value.

Optionally, the apparatus further includes:
a calculation module, which is configured to assign different base values and weight values to the real-time change amplitude of the impedance value and the real-time change amplitude of the temperature value when the real-time change information includes the real-time change trend and the real-time change amplitude of the impedance value and the real-time change trend and the real-time change amplitude of the temperature value, and calculate an adjustment amplitude according to the base value and the weight value in a process of dynamical adjustment of the perfusion amount of the syringe pump, wherein the weight value corresponding to the real-time change amplitude of the temperature value is smaller than the weight value corresponding to the real-time change amplitude of the impedance value.

Optionally, the apparatus further includes:
a first emergency control module, which is configured to control the syringe pump to stop the perfusion operation when the detected temperature value is greater than a first abnormal value or less than a second abnormal value for more than a first preset duration, wherein the first preset duration is greater than or equal to zero.

Optionally, the apparatus further includes:
a second emergency control module, which is configured to control the syringe pump to stop the perfusion operation when the detected impedance value is greater than a third abnormal value or less than a fourth abnormal value for more than a second preset duration, wherein the second preset duration is greater than or equal to zero.

For a specific process of the above modules to realize their respective functions, reference is made to relevant contents in the embodiments shown in FIG. 4 to FIG. 9, which will be omitted here.

In the embodiment of the present application, when the ablation task is triggered, the syringe pump is controlled to perform the perfusion operation on the ablation object, and the impedance value and/or temperature value of the ablation object are acquired in real time; and then the acquired impedance value and/or temperature value is analyzed, and the perfusion amount of the syringe pump is dynamically adjusted according to real-time change information of the impedance value and/or temperature value obtained from the analysis. Accordingly, automatic dynamic adjustment of the perfusion amount of the syringe pump is realized based on the analysis of changes of the impedance value and/or temperature value of the ablation object during the process of executing the ablation task. Since the perfusion amount of the syringe pump is adjusted automatically and dynamically along with the changing of the impedance value and/or temperature value of the ablation object, operation delays and errors caused by manual determination can be reduced, and the timeliness and the accuracy of liquid perfusion during the process of executing the ablation task can be improved. Accordingly, the injury of the ablation operation to the ablation object is reduced, and the safety of the radio frequency ablation treatment is improved.

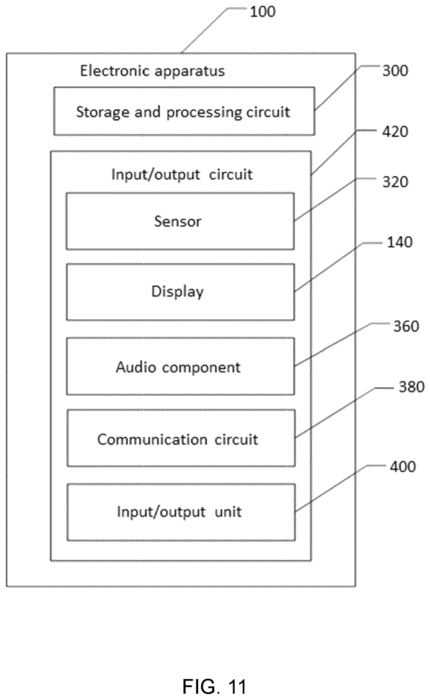

Referring to FIG. 11, which is a schematic diagram showing a hardware structure of an electronic apparatus provided by an embodiment of the present application.

Exemplarily, the electronic apparatus may be any one of various types of computer system devices that are non-movable or movable or portable and perform wireless or wired communication. Particularly, the electronic apparatus may be a desktop computer, a server, a mobile phone or a smart phone (for example, an iPhone-TM-based phone, an Android-TM-based phone), a portable game device (for example, Nintendo DS TM, PlayStation Portable TM, Gameboy Advance TM, iPhone TM), a laptop computer, a PDA, a portable Internet device, a music player and a data storage device, and other handheld devices such as watches, earphones, pendants, and earphones. The electronic apparatus may further be other wearable devices (for example, electronic glasses, electronic clothes, electronic bracelets, electronic necklaces, electronic tattoos, electronic apparatuses or head-mounted devices (HMD) of the smart watches).

The electronic apparatus may be any one of multiple electronic apparatuses, including but not limited to a cellular phone, a smart phone, other wireless communication devices, a personal digital assistant, an audio player, other media players, a music recorder, a video recorder, a camera, other media recorders, a radio, a medical device, a vehicle transportation instrument, a calculator, a programmable remote control, a pager, a laptop computer, a desktop computer, a printer, a netbook computer, a personal digital assistant (PDA), a portable multimedia player (PMP), a motion picture experts group (MPEG-1 or MPEG-2) audio layer 3 (MP3) player, a portable medical device and a digital camera, and a combination thereof.

In some cases, the electronic apparatus may perform multiple functions (for example, playing music, displaying videos, storing pictures, and receiving and transmitting phone calls). If desired, the electronic apparatus may be a portable device such as a cell phone, a media player, other handheld devices, a wrist watch device, a pendant device, an earpiece device, or other compact portable devices.

As shown in FIG. 11, the electronic apparatus 100 may include a control circuit, wherein the control circuit may include a storage and processing circuit 300. The storage and processing circuit 300 may include a memory, such as a hard disk drive memory, a non-transitory or non-volatile memory (such as a flash memory or other electronically programmable restricted deletion memory configured to form a solid-state drive), and a volatile memory (for example, a static or dynamic random access memory and the like), and the like, which are not limited in the embodiment of the present application. The processing circuit in the storage and processing circuit 300 may be configured to control the operation of the electronic apparatus 100. The processing circuit may be implemented based on one or more microprocessors, microcontrollers, digital signal processors, baseband processors, power management units, audio codec chips, application specific integrated circuits, display driver integrated circuits, and the like.

The storage and processing circuit 300 may be configured to execute software in the electronic apparatus 100, such as an Internet browsing application, a Voice over Internet Protocol (VOIP) telephone calling application, an email application, a media playback application, an operating system function, and the like. The software may be configured to perform some control operations, for example, image capture based on a camera, ambient light measurement based on an ambient light sensor, proximity sensor measurement based on a proximity sensor, and information display function realized based on a status indicator such as a status indicator lamp of a LED, touch event detection based on a touch sensor, a function associated with displaying information on multiple (for example, layered) displays, an operation associated with performing a wireless communication function, an operation associated with acquiring and generating an audio signal, a control operation associated with acquiring and processing of button press event data, and other functions in the electronic apparatus 100, which are not limited in the embodiment of the present application.

Further, the memory stores an executable program code, and a processor coupled with the memory calls the executable program code stored in the memory to perform the above perfusion control method for syringe pump described in the embodiments shown in FIG. 4 to FIG. 9.

Among them, the executable program code includes various modules in the perfusion control apparatus for syringe pump described in the embodiment shown in FIG. 10, for example, the control module 201, the analysis module 202 and the adjustment module 203.

The electronic apparatus 100 may further include an input/output circuit 420. The input/output circuit 420 may be configured to enable the electronic apparatus 100 to input and output data, that is, to allow the electronic apparatus 100 to receive data from an external device and further allow the electronic apparatus 100 to output data from the electronic apparatus 100 to the external device. The input/output circuit 420 may further include a sensor 320. The sensor 320 may include an ambient light sensor, a proximity sensor based on light and capacitive, and a touch sensor (for example, a light-based touch sensor and/or a capacitive touch sensor, wherein the touch sensor may be a part of a touch display screen, or may be independently used as a touch sensor), an acceleration sensor, other sensors and the like.

The input/output circuit 420 may further include one or more displays, such as a display 140. The display 140 may include one or a combination of a liquid crystal display, an organic light emitting diode display, an electronic ink display, a plasma display, and a display using other display technologies. The display 140 may include a touch sensor array (i.e., the display 140 may be a touch display screen). The touch sensor can be a capacitive touch sensor formed by an array of transparent touch sensor electrodes (for example, indium tin oxide (ITO) electrodes), or a touch sensor formed using other touch technologies, such as sonic touch, pressure-sensitive touch, resistance touch, optical touch, and the like, which are not limited in the embodiment of the present application.

The electronic apparatus 100 may further include an audio component 360. The audio component 360 may be configured to provide audio input and output functions for the electronic apparatus 100. The audio component 360 in the electronic apparatus 100 may include a speaker, a microphone, a buzzer, a tone generator, and other components for generating and detecting sounds.

The communication circuit 380 may be configured to provide the electronic apparatus 100 with the ability to communicate with an external device. The communication circuit 380 may include an analog and digital input/output interface circuit, and a wireless communication circuit based on a radio frequency signal and/or an optical signal. The wireless communication circuit in the communication circuit 380 may include a radio frequency transceiver circuit, a power amplifier circuit, a low noise amplifier, a switch, a filter and an antenna. For example, the wireless communication circuit in the communication circuit 380 may include a circuit for supporting near field communication (NFC) by transmitting and receiving a near-field coupled electromagnetic signal. For example, the communication circuit 380 may include a near-field communication antenna and a near-field communication transceiver. The communication circuit 380 may further include a cellular phone transceiver and an antenna, a wireless local area network transceiver circuit and an antenna, and the like.

The electronic apparatus 100 may further include a battery, a power management circuit and other input/output units 400. The input/output unit 400 may include a button, a joystick, a click wheel, a scroll wheel, a touch pad, a keypad, a keyboard, a camera, a light emitting diode, and other status indicators.

The user may input a command through the input/output circuit 420 to control the operation of the electronic apparatus 100, and may use the output data of the input/output circuit 420 to realize the reception of status information and other outputs from the electronic apparatus 100.

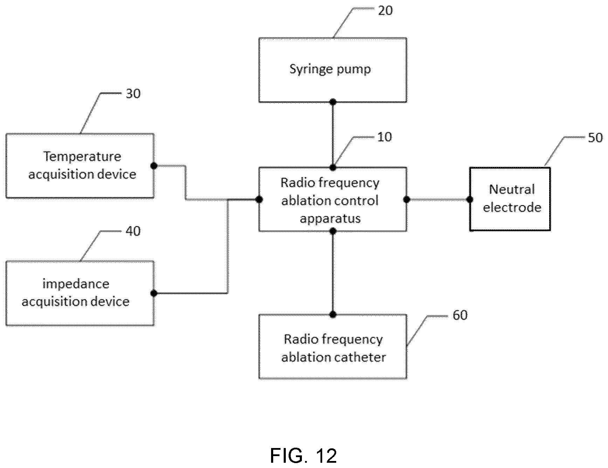

Further, as shown in FIG. 12, embodiments of the present application further provide a radio frequency ablation system. The radio frequency ablation system includes a radio frequency ablation control apparatus 10, a syringe pump 20, a temperature acquisition device 30, an impedance acquisition device 40, a neutral electrode 50 and a radio frequency ablation catheter 60.

As shown in FIG. 1, the radio frequency ablation catheter 60, the temperature acquisition device 30, the impedance acquisition device 40 and the neutral electrode 50 are electrically connected to the radio frequency ablation control apparatus 10. The radio frequency ablation control apparatus 10 is further electrically coupled to the syringe pump 20.

The radio frequency ablation control apparatus 10 is configured to perform steps of the perfusion control method for syringe pump according to the embodiments shown in FIG. 4 to FIG. 9.

The radio frequency ablation catheter 60 is configured to perform an ablation operation on the ablation object according to a control instruction of the radio frequency ablation control apparatus 10.

The temperature acquisition device 30 is configured to acquire a temperature value of the ablation object and transmit it to the radio frequency ablation control apparatus 10.

The impedance acquisition device 40 is configured to acquire an impedance value of the ablation object and transmit it to the radio frequency ablation control apparatus 10.

Further, embodiments of the present application further provide a computer-readable storage medium. The computer-readable storage medium may be provided in the electronic apparatus of each of the foregoing embodiments, and the computer-readable storage medium may be a memory in the storage and processing circuit 300 of the embodiment as shown in FIG. 11. A computer program is stored on the computer-readable storage medium, and is executed by the processor to realize the perfusion control method for syringe pump described in the foregoing embodiments as shown in FIG. 4 to FIG. 9. Further, the computer-storable storage medium may further be a U disk, a mobile hard disk, a read-only memory (ROM), a RAM, a magnetic disk or an optical disk, and other various media that may store the program code.

In the several embodiments provided in the present application, it should be understood that the disclosed apparatus and method may be implemented in other ways. For example, the embodiments of the apparatus described above are merely illustrative. For example, the division of the modules is only a logical function division, or other divisions in practical implementations, for example, multiple modules or components may be combined or may be integrated into another system, or some features may be ignored or not performed. In addition, the displayed or discussed mutual coupling or direct coupling or communication connection may be indirect coupling or communication connection through some interfaces, apparatuses or modules, and may be in electrical, mechanical or other forms.

The modules described as separate components may or may not be physically separated, and the components displayed as modules may or may not be physical modules, that is, they may be located in one place, or they may be distributed onto multiple network modules. Some or all of the modules may be selected according to actual needs to achieve the objectives of the solutions of the embodiments.

In addition, the functional modules in the various embodiments of the present application may be integrated into one processing module, or each module may exist alone physically, or two or more modules may be integrated into one module. The above-mentioned integrated modules may be implemented in the form of hardware or a software functional module.

If the integrated module is implemented in the form of the software function module and sold or used as an independent product, it may be stored in a computer-readable storage medium. Based on such understanding, the technical solution of the present application substantively, or a part thereof making a contribution to the prior art, or all or part of the technical solution may be embodied in the form of a software product stored in a readable storage medium, and the readable storage medium includes several instructions to enable a computer device (which may be a personal computer, a server, or a network device) to execute all or part of the steps of the methods described in the various embodiments of the present application. The above-mentioned readable storage medium includes a U disk, a mobile hard disk, a ROM, a RAM, a magnetic disk or an optical disk, and other media that may store the program code.

It should be noted that for the foregoing method embodiments, for simplicity of description, they are all expressed as a series of action combinations, but those skilled in the art should appreciate that the present application is not limited by the described sequence of actions. Because according to the present application, some steps may be performed in other sequences or simultaneously, and secondly, those skilled in the art should further appreciate that the embodiments described in the specification are all preferred embodiments, and the involved actions and modules are not necessarily all required by the present application.

In the above-mentioned embodiments, descriptions of the embodiment have particular emphasis respectively. For parts that are not described in detail in a certain embodiment, reference may be made to related descriptions of other embodiments.

The above is a description of the perfusion control method, apparatus and system for syringe pump, and the computer-readable storage medium according to the present application. For those skilled in the art, according to the ideas of the embodiments of the present application, changes may be made to specific implementations and application scopes. In summary, the content of this specification should not be construed as a limitation on the present application.

## Claims

1. A perfusion control method for syringe pump, comprising:
when an ablation task is triggered, controlling the syringe pump to execute a perfusion operation on an ablation object, and acquiring an impedance value and/or a temperature value of the ablation object in real time;
analyzing the acquired impedance value and/or temperature value to obtain real-time change information of the impedance value and/or temperature value; and
adjusting a perfusion amount of the syringe pump dynamically according to the real-time change information obtained from the analysis.

2. The method according to claim 1, wherein the real-time change information comprises a real-time change trend and a real-time change amplitude.

3. The method according to claim 2, wherein adjusting the perfusion amount of the syringe pump dynamically according to the real-time change information obtained from the analysis comprising:
determining a current ablation stage according to the real-time change trend and the real-time change amplitude of the impedance value, and adjusting the perfusion amount of the syringe pump according to a target adjustment logic corresponding to the current ablation stage.

4. The method according to claim 3, wherein when the real-time acquired data comprises at least the impedance value, determining the current ablation stage according to the real-time change trend and the real-time change amplitude of the impedance value and adjusting the perfusion amount of the syringe pump according to the target adjustment logic corresponding to the current ablation stage comprising:
when the syringe pump starts to perfuse liquid into the ablation object, determining the beginning of a first ablation stage, and adjusting the perfusion amount of the syringe pump according to a first target adjustment logic based on the real-time change trend and the real-time change amplitude of the impedance value;
when the impedance value presents a first real-time change trend and reaches a first real-time change amplitude in the first ablation stage, determining the beginning of a second ablation stage, and adjusting the perfusion amount of the syringe pump according to a second target adjustment logic;
when the impedance value presents a second real-time change trend and reaches a second real-time change amplitude in the second ablation stage, determining the beginning of a third ablation stage, and adjusting the perfusion amount of the syringe pump according to a third target adjustment logic; and
when the impedance value presents a third real-time change trend and reaches a third real-time change amplitude in the third ablation stage, determining the beginning of a fourth ablation stage, and adjusting the perfusion amount of the syringe pump according to a fourth target adjustment logic.

5. The method according to claim 4, wherein when the real-time acquired data comprises the impedance value and the temperature value, adjusting the perfusion amount of the syringe pump according to the first target adjustment logic based on the real-time change trend and the real-time change amplitude of the impedance value, comprising:
judging whether the impedance value presents a rising trend and reaches a preset first increase:
when the impedance value presents the rising trend but does not reach the preset first increase, judging whether the impedance value and the temperature value are stable:
when the impedance value and the temperature value are stable, controlling the syringe pump to decrease the perfusion amount according to a preset first adjustment amplitude, and returning to perform the step of judging whether the impedance value presents the rising trend and reaches the preset first increase; and
when the impedance value presents the rising trend and reaches the preset first increase, controlling the syringe pump to increase the perfusion amount according to a preset second adjustment amplitude.

6. The method according to claim 4, wherein
when the impedance value presents the first real-time change trend and reaches the first real-time change amplitude in the first ablation stage, determining the beginning of the second ablation stage and adjusting the perfusion amount of the syringe pump according to the second target adjustment logic, comprising:
determining the beginning of the second ablation stage when the impedance value presents the rising trend and reaches the preset second increase in the first ablation stage; and
controlling the syringe pump to increase the perfusion amount according to a preset third adjustment amplitude.

7. The method according to claim 4, whereinwhen the impedance value presents the second real-time change trend and reaches the second real-time change amplitude in the second ablation stage, determining the beginning of the third ablation stage and adjusting the perfusion amount of the syringe pump according to the third target adjustment logic, comprising:
determining the beginning of the third ablation stage when the impedance value presents the rising trend and reaches the preset third increase in the second ablation stage; and
controlling the syringe pump to increase the perfusion amount according to a preset fourth adjustment amplitude.

8. The method according to claim 4, wherein when the impedance value presents third real-time change trend and reaches the third real-time change amplitude in the third ablation stage, determining the beginning of the fourth ablation stage and adjusting the perfusion amount of the syringe pump according to the fourth target adjustment logic, comprising:
determining the beginning of the fourth ablation stage when the impedance value presents the rising trend and reaches the preset fourth increase in the third ablation stage, and controlling the syringe pump to increase the perfusion amount according to a preset fifth adjustment amplitude; or,
determining the beginning of the fourth ablation stage when the impedance value shows a falling trend and reaches a preset first decrease, and controlling the syringe pump to decrease the perfusion amount to an initial value.

9. The method according to claim 2, wherein analyzing the acquired impedance value and/or temperature value to obtain the real-time change information of the impedance value and/or temperature value and adjusting the perfusion amount of the syringe pump dynamically according to the real-time change information obtained from the analysis comprises:
analyzing whether the impedance value or the temperature value presents a rising trend or a falling trend:
when the impedance value or the temperature value presents the rising trend, controlling the syringe pump to increase a perfusion flow rate according to a preset increase and a trend change of the impedance value or the temperature value; and
when the impedance value or the temperature value presents the falling trend, controlling the syringe pump to decrease the perfusion flow rate according to a preset decrease and the trend change of the impedance value or the temperature value.

10. The method according to claim 9, wherein analyzing whether the impedance value or the temperature value presents the rising trend or the falling trend: when the impedance value or the temperature value presents the rising trend, controlling the syringe pump to increase the perfusion flow rate according to the preset increase and the trend change of the impedance value or the temperature value; and when the impedance value or the temperature value presents the falling trend, controlling the syringe pump to decrease the perfusion flow rate according to the preset decrease and the trend change of the impedance value or the temperature value compris:
judging whether the impedance value or the temperature value increases according to the real-time change information:
when the impedance value or the temperature value increases, controlling the syringe pump to increase the perfusion flow rate according to a preset first increase, and returning to perform the step of judging whether the impedance value or the impedance value increases according to the real-time change information;
when the impedance value or the temperature value does not increase, judging whether the impedance value or the temperature value decreases according to the real-time change information:
when the impedance value or the temperature value decreases, controlling the syringe pump to decrease the perfusion flow rate according to a preset first decrease, and returning to perform the step of judging whether the impedance value or the temperature value decreases according to the real-time change information; and
when the impedance value or the temperature value does not decrease, returning to perform the step of judging whether the impedance value or the temperature value increases according to the real-time change information.

11. The method according to claim 9, wherein controlling the syringe pump to increase the perfusion flow rate according to the preset increase and the trend change in the impedance value or the temperature value, comprising:
when the impedance value or the temperature value is greater than a preset first threshold, controlling the syringe pump to increase the perfusion flow rate according to a preset second increase;
judging whether the impedance value or the temperature value continues to increase:
when the impedance value or the temperature value continues to increase, calculating a third increase according to the second increase and the number of adjustments to the perfusion flow rate;
controlling the syringe pump to increase the perfusion flow rate according to the third increase and returning to perform the step of judging whether the impedance value or the temperature value continues to increase until the perfusion flow rate increases to a preset maximum flow rate;
when the impedance value or the temperature value does not continue to increase, judging whether the impedance value or the temperature value is less than the preset first threshold:
when the impedance value or the temperature value is not less than the preset first threshold, controlling the syringe pump to increase the perfusion flow rate according to the second increase until the perfusion flow rate increases to the preset maximum flow rate; and
when the impedance value or the temperature value is less than the preset first threshold, returning to perform the step of judging whether the impedance value or the temperature value presents the rising trend or the falling trend according to the real-time change information obtained from the analysis.

12. The method according to claim 9, whereincontrolling the syringe pump to decrease the perfusion flow rate according to the preset decrease and the trend change in the impedance value or the temperature value, comprising:
when the impedance value or the temperature value is less than a preset second threshold, controlling the syringe pump to decrease the perfusion flow rate of the syringe pump according to a preset second decrease;
judging whether the impedance value or the temperature value continues to decrease:
when the impedance value or the temperature value continues to decrease, calculating a third decrease according to the second decrease and the number of adjustments to the perfusion flow rate;
controlling the syringe pump to decrease the perfusion flow rate according to the third decrease and returning to perform the step of judging whether the impedance value or the temperature value continues to decrease until the perfusion flow rate decreases a preset minimum flow rate;
when the impedance value or the temperature value does not continue to decrease, judging whether the impedance value or the temperature value is greater than the preset second threshold:
when the impedance value or the temperature value is not greater than the preset second threshold, controlling the syringe pump to decrease the perfusion flow rate according to the second decrease and returning to perform the step of judging whether the temperature value or the temperature value is greater than the preset second threshold until the perfusion flow rate decreases to the preset minimum flow rate; and
when the impedance value or the temperature value is greater than the preset second threshold, returning to perform the step of judging whether the impedance value or the temperature value presents the rising trend or the falling trend according to the real-time change information obtained from the analysis.

13. The method according to any one of claims 1 to 12, wherein acquiring the impedance value and/or temperature value of the ablation object in real time comprising:
acquiring an impedance sample value and/or a temperature sample value of the ablation object in real time; and
filtering the acquired impedance sample value and/or temperature sample value and taking the filtered impedance sample value and/or temperature sample value as the impedance value and/or temperature value.

14. The method according to any one of claims 1 to 12, whereinacquiring the impedance value and/or temperature value of the ablation object in real time, comprising:
acquiring an impedance sample value and/or a temperature sample value of the ablation object in real time;
filtering the acquired impedance sample value and/or temperature sample value;
judging whether the filtered impedance sample value and/or temperature sample value exceed/exceeds a preset warning value range:
when the filtered impedance sample value and/or temperature sample value exceed/exceeds the preset warning value range, output alarm information; and
when the filtered impedance sample value and/or temperature sample value do/does not exceed the preset warning value range, taking the minimum value or an average value of the filtered impedance sample value and/or temperature sample value within a preset period as the impedance value and/or temperature value.

15. The method according to any one of claims 2 to 12, wherein when the real-time change information comprises a real-time change trend and a real-time change amplitude of the impedance value and a real-time change trend and a real-time change amplitude of the temperature value, adjusting the perfusion amount of the syringe pump dynamically according to the real-time change information obtained from the analysis, comprising:
analyzing whether the real-time change trend and the real-time change amplitude of the impedance value and the real-time change trend and the real-time change amplitude of the temperature value meet an adjustment condition:
when the real-time change trend and the real-time change amplitude of the impedance value and the real-time change trend and the real-time change amplitude of the temperature value meet the adjustment condition, adjusting the perfusion amount of the syringe pump dynamically according to the real-time change trend and the real-time change amplitude of the impedance value.

16. The method according to any one of claims 2 to 12, wherein when the real-time change information comprises a real-time change trend and a real-time change amplitude of the impedance value and a real-time change trend and a real-time change amplitude of the temperature value, further comprising:
assigning different base values and weight values to the real-time change amplitude of the impedance value and the real-time change amplitude of the temperature value; and
calculating an adjustment amplitude according to the base values and the weight values during the process of dynamical adjustment of the perfusion amount of the syringe pump;
wherein the weight value corresponding to the real-time change amplitude of the temperature value is smaller than the weight value corresponding to the real-time change amplitude of the impedance value.

17. The method according to any one of claims 1 to 12, further comprising:
when the detected temperature value is greater than a first abnormal value or less than a second abnormal value for more than a first preset duration, controlling the syringe pump to stop the perfusion operation, wherein the first preset duration is greater than or equal to zero;

18. The method according to any one of claims 1 to 12, further comprising:
when the detected impedance value is greater than a third abnormal value or less than a fourth abnormal value for more than a second preset duration, controlling the syringe pump to stop the perfusion operation, wherein the second preset duration is greater than or equal to zero.

19. A perfusion control apparatus for syringe pump, comprising:
a control module configured for controlling the syringe pump to execute a perfusion operation on an ablation object when an ablation task is triggered, and acquiring an impedance value and/or a temperature value of the ablation object in real time;
an analysis module configured for analyzing the acquired impedance value and/or temperature value to obtain real-time change information of the impedance value and/or temperature value; and
an adjustment module configured for adjusting the perfusion amount of the syringe pump dynamically according to the real-time change information obtained from the analysis.

20. An electronic apparatus, comprising:
a non-transitory memory and a processor, wherein
the non-transitory memory stores an executable program code;
the processor is electrically coupled to the non-transitory memory, a temperature acquisition device and an impedance acquisition device; and
the processor calls the executable program code stored in the non-transitory memory to execute the perfusion control method for syringe pump according to any one of claims 1 to 19.

21. A perfusion control system for syringe pump, comprising a syringe pump, a temperature acquisition device and an impedance acquisition device, wherein
the syringe pump comprises a controller, a syringe, a push rod and a driving device, wherein
the controller is electrically coupled to the driving device, the temperature acquisition device and the impedance acquisition device, for executing the steps of the perfusion control method for syringe pump according to any one of claims 1 to 18;
the driving device is configured for driving the push rod to move along a desired direction with a desired speed pointed by the control instruction of the controller so as to control and adjust the perfusion amount of the syringe;
the temperature acquisition device is configured for acquiring a temperature value of the ablation object and transmitting it to the controller; and
the impedance acquisition device is configured for acquiring an impedance value of the ablation object and transmitting it to the controller.

22. A radio frequency ablation system, comprising a radio frequency ablation control apparatus, a radio frequency ablation catheter, a neutral electrode, a syringe pump, a temperature acquisition device and an impedance acquisition device, wherein
the radio frequency ablation control apparatus is configured for executing steps of the perfusion control method for syringe pump according to any one of claims 1 to 18;
the radio frequency ablation catheter is configured for executing an ablation operation on the ablation object according to a control instruction of the radio frequency ablation control apparatus.
the temperature acquisition device is configured for acquiring a temperature value of the ablation object and transmitting it to the radio frequency ablation control apparatus; and
the impedance acquisition device is configured for acquiring an impedance value of the ablation object and transmitting it to the radio frequency ablation control apparatus.

23. A non-transitory computer-readable storage medium on which a computer program is stored, wherein the computer program, when executed by a processor, implements the perfusion control method for syringe pump according to any one of claims 1 to 18.
